# EUROPÄISCHE PATENTSCHRIFT

(11) **EP 0 764 637 B1**
(45) Veröffentlichungstag und Bekanntmachung des Hinweises auf die Patenterteilung: **30.06.1999**
(21) Anmeldenummer: 96114713.9
(22) Anmeldetag: 13.09.1996
(51) Int. Cl.: C07C 323/60, A61K 31/275

(54) **2-Cyano-3-mercaptocrotonsäureamide**
2-Cyano-3-mercaptocrotonamides
Amides 2-cyano-3-mercaptocrotoniques

(30) Priorität: 19.09.1995 DE 19534649
(43) Veröffentlichungstag der Anmeldung: 26.03.1997
(73) Patentinhaber: HOECHST AKTIENGESELLSCHAFT, 65929 Frankfurt am Main (DE)
(72) Erfinder: Kämmerer, Friedrich-Johannes, Dr., 65239 Hochheim (DE); Schleyerbach, Rudolf, Dr., 65719 Hofheim (DE); Thorwart, Werner, Dr., 65239 Hochheim (DE)

(56) Entgegenhaltungen:
- ARCHIV DER PHARMAZIE UND BERICHTE DER DEUTSCHEN PHARMAZEUTISCHEN GESELLSCHAFT, Bd. 301, Nr. 1, Januar 1968, WEINHEIM DE, Seiten 601-610, XP000604529 K. HARTKE ET AL: "Thioacylierung von Malonsäurederivaten mit Dithio- und Thionestern"
- JOURNAL OF ORGANIC CHEMISTRY, Bd. 49, Nr. 1, 1984, EASTON US, Seiten 74-78, XP000644573 M. YOKOYAMA ET AL: "S,N Double Rearrangement. 3. Reaction Mechanism"

## Beschreibung

Die Erfindung betrifft 2-Cyano-3-mercaptocrotonsäureamidderivate, Verfahren zu ihrer Herstellung und Verwendung derselben als Arzneimittel.

Einige der erfindungsgemäße verwendbaren Verbindungen sind bereits beschrieben worden, doch über ihre Verwendung als Arzneimittel ist nichts bekannt (Hartke et al., Arch Pharm, (1968) 301 (8), Seiten 601-610; Yokoyama et al., J Org Chem, (1984) 49, Seiten 74-78).

Die Erfindung betrifft eine Verbindung der Formel I, 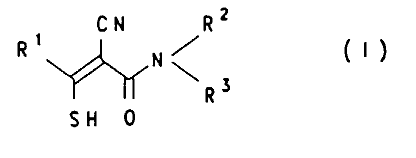 und/oder eine gegebenenfalls stereoisomere Form der Verbindung der Formel I und/oder ein physiologisch verträgliches Salz der Verbindung der Formel I, wobei
- R¹: für
a) Wasserstoffatom,
b) (C₁-C₁₇)-Alkyl,
c) (C₁-C₄)-Alkyl, ein- oder mehrfach substituiert durch Fluor, Chlor, Brom oder Jod,
d) Phenyl,
e) Phenyl, ein- oder mehrfach substituiert durch
   1) Fluor, Chlor, Brom oder Jod,
   2) Nitro,
   3) Cyano,
   4) (C₁-C₄)-Alkyl,
   5) (C₁-C₄)-Alkyl, ein- oder mehrfach substituiert durch Fluor, Chlor, Brom oder Jod,
   6) (C₁-C₄)-Alkoxy,
   7) (C₁-C₄)-Alkoxy, ein- oder mehrfach substituiert durch Fluor, Chlor, Brom oder Jod,
f) Benzyl,
g) (C₃-C₇)-Cycloalkyl,
h) Alkenyl mit 2 oder 3 C-Atomen oder
i) Alkinyl mit 2 oder 3 C-Atomen, steht,
- R²: für
a) Wasserstoffatom,
b) (C₁-C₄)-Alkyl,
c) Phenyl,
d) Phenyl-(C₁-C₂)-alkyl oder
e) Alkenyl mit 2 bis 3 C-Atomen, steht, oder
- R³: für
a) einen ein-, zwei- oder dreikernigen, ungesättigten heterocyclischen Rest mit 3 bis 13 C-Atomen und 1 bis 4 Heteroatomen aus der Gruppe Sauerstoff, Schwefel und Stickstoff steht, wovon im Ringsystem höchstens eines der Heteroatome von Stickstoff verschieden ist, und unsubstituiert ist oder ein- oder mehrfach substituiert ist durch
   1) Fluor, Chlor, Brom oder Jod,
   2) (C₁-C₄)-Alkyl,
   3) (C₁-C₄)-Alkyl, ein- oder mehrfach substituiert durch Fluor, Chlor, Brom oder Jod,
   4) (C₁-C₄)-Alkoxy,
   5) (C₁-C₄)-Alkoxy, ein- oder mehrfach substituiert durch Fluor, Chlor, Brom oder Jod,
   6) Nitro,
   7) Hydroxy,
   8) Carboxy,
   9) Carbamoyl oder
   10) Oxogruppe,
b) einen Rest der Formel II, 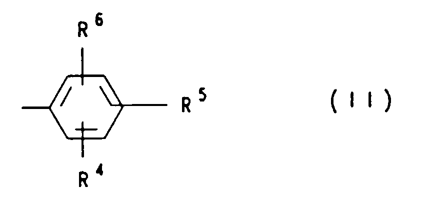 in der R⁴, R⁵ und R⁶ gleich oder verschieden sein können und für
   1) Wasserstoffatom,
   2) (C₁-C₄)-Alkyl,
   3) (C₁-C₄)-Alkyl, ein- oder mehrfach substituiert durch Fluor, Chlor, Brom oder Jod,
   4) in der R⁴ für Wasserstoffatom und R⁵ und R⁶ gemeinsam mit dem Phenylring der Formel II einen Naphthalinring bilden,
   5) in der R⁴ für Wasserstoffatom und R⁵ und R⁶ einen Methylendioxyrest bilden,
   6) (C₁-C₄)-Alkoxy,
   7) (C₁-C₄)-Alkoxy, ein- oder mehrfach substituiert durch Fluor, Chlor, Brom oder Jod,
   8) (C₁-C₄)-Alkylmercapto,
   9) (C₁-C₄)-Alkylmercapto, ein- oder mehrfach substituiert durch Fluor, Chlor, Brom oder Jod,
   10) Fluor, Chlor, Brom oder Jod,
   11) Nitro,
   12) Cyano,
   13) Hydroxy,
   14) Carboxy,
   15) (C₁-C₄)-Alkylsulfonyl,
   16) Carbalkoxy, mit 1 bis 3 C-Atomen in der Alkylkette,
   17) Benzoyl,
   18) Benzoyl, ein- oder mehrfach substituiert durch
      18.1 Fluor, Chlor, Brom oder Jod,
      18.2 (C₁-C₄)-Alkyl oder
      18.3 (C₁-C₄)-Alkoxy,
   19) Phenyl,
   20) Phenyl, ein- oder mehrfach substituiert durch
      20.1 (C₁-C₄)-Alkoxy,
      20.2 Fluor, Chlor, Brom oder Jod oder
      20.3 (C₁-C₄)-Alkyl,
   21) Phenoxy, oder
   22) Phenoxy, ein- oder mehrfach substituiert durch (C₁-C₃)-Alkoxy, ein- oder mehrfach substituiert durch
      22.1 Fluor, Chlor, Brom oder Jod,
      22.2 (C₁-C₃)-Alkyl, ein- oder mehrfach substituiert durch Fluor, Chlor, Brom oder Jod, oder
      22.3 Fluor, Chlor, Brom oder Jod,
c) einen Rest der Formel III steht,

   -(CH₂)ₙ-COOR¹⁰ (III)

   in der R¹⁰ für
      1) Wasserstoffatom oder
      2) (C₁-C₄)-Alkyl und
   n für eine ganze Zahl von 1 bis 12 steht, oder
d) R² und R³ bilden zusammen mit dem Stickstoffatom, an das sie gebunden sind, einen 4- bis 7-gliedrigen Ring, der unsubstituiert oder substituiert ist durch Carbonyl am N-Atom benachbarten C-Atom, oder
e) R² und R³ bilden zusammen mit dem Stickstoffatom, an das sie gebunden sind, einen 5- bis 6-gliedrigen Ring der Formel IV 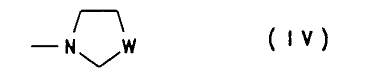 in der W für
   1)

      -CH₂-,
   2)

      -CH₂-CH₂-,
   3) 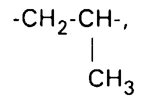
   4) 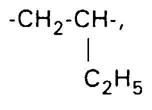
   5) 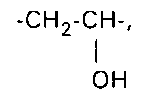
   6)

      -CH₂-O-

      oder
   7)

      -CH₂-S-

      steht.

Bevorzugt ist eine Verbindung der Formel I und/oder ein physiologisch verträgliches Salz der Verbindung der Formel I und/oder eine gegebenenfalls stereoisomere Form der Verbindung der Formel I, wobei
- R¹: für
a) Wasserstoffatom,
b) (C₁-C₆)-Alkyl,
c) (C₁-C₄)-Alkyl, ein- oder mehrfach substituiert durch Fluor, Chlor, Brom oder Jod,
d) Phenyl,
e) Alkenyl mit 2 oder 3 C-Atomen oder
f) (C₃-C₄)-Cyloalkyl, steht
- R²: für
a) Wasserstoffatom,
b) (C₁-C₄)-Alkyl,
c) Benzyl oder
d) Alkenyl mit 2 bis 3 C-Atomen steht,
- R³: für
a) Pyridyl, ein- oder mehrfach substituiert durch
   1) Wasserstoffatom,
   2) Fluor, Chlor, Brom oder Jod,
   3) Nitro,
   4) (C₁-C₃)-Alkyl oder
   5) (C₁-C₃)-Alkoxy,
b) einen Rest der Formel II, in der R⁴, R⁵ und R⁶ gleich oder verschieden sein können und für
   1) Wasserstoffatom,
   2) (C₁-C₃)-Alkyl,
   3) (C₁-C₃)-Alkyl, ein oder mehrfach substituiert durch Fluor, Chlor, Brom oder Jod,
   4) in der R⁴ für Wasserstoffatom steht und R⁵ und R⁶ einen Methylendioxyrest bilden,
   5) (C₁-C₃)-Alkoxy,
   6) (C₁-C₃)-Alkoxy, ein- oder mehrfach substituiert durch Fluor, Chlor, Brom oder Jod,
   7) (C₁-C₃)-Alkylmercapto,
   8) (C₁-C₃)-Alkylmercapto, ein- oder mehrfach substituiert durch Fluor, Chlor, Brom oder Jod,
   9) Fluor, Chlor, Brom oder Jod,
   10) Nitro,
   11) Cyano,
   12) (C₁-C₃)-Alkylsulfonyl,
   13) Benzoyl,
   14) Benzoyl, ein- oder mehrfach substituiert durch
      14.1 Fluor, Chlor, Brom oder Jod
      14.2 (C₁-C₃)-Alkyl oder
      14.3 (C₁-C₃)-Alkoxy,
   15) Phenoxy, oder
   16) Phenoxy, ein- oder mehrfach substituiert durch
      16.1 (C₁-C₃)-Alkoxy, ein- oder mehrfach substituiert durch Fluor, Chlor, Brom oder Jod,
      16.2 Fluor, Chlor, Brom oder Jod,
      16.3 (C₁-C₃)-Alkyl, ein- oder mehrfach substituiert durch Fluor, Chlor, Brom, oder Jod
c) einen Rest der Formel III steht und in der R¹⁰ für
   1) Wasserstoffatom,
   2) (C₁-C₄)-Alkyl und
   n für eine ganze Zahl von 1 bis 12 steht, oder
d) R² und R³ bilden zusammen mit dem Stickstoff, an das sie gebunden sind, einen 4- bis 7-gliedrigen Ring, der durch Carbonyl am N-Atom benachbarten C-Atom substituiert ist.

Insbesondere bevorzugt ist eine Verbindung der Formel I und/oder ein physiologisch verträgliches Salz der Verbindung der Formel I und/oder eine gegebenenfalls stereoisomere Form der Verbindung der Formel I, wobei
- R¹: für (C₁-C₆)-Alkyl steht,
- R²: für Wasserstoffatom steht,
- R³: für
a) Pyridyl, ein- oder mehrfach substituiert durch Fluor, Chlor, Brom oder Jod, oder
b) einen Rest der Formel II, in der R⁴, R⁵ und R⁶ gleich oder verschieden sein können und für
   1) Wasserstoffatom oder,
   2) (C₁-C₃)-Alkyl,
   3) (C₁-C₃)-Alkyl, ein- oder mehrfach substituiert durch Fluor, Chlor, Brom oder Jod,
   4) in der R⁴ für Wasserstoffatom und R⁵ und R⁶ einen Methylendioxyrest bilden,
   5) (C₁-C₃)-Alkoxy,
   6) (C₁-C₃)-Alkoxy, ein- oder mehrfach substituiert durch Fluor, Chlor, Brom oder Jod,
   7) Fluor, Chlor, Brom oder Jod,
   8) Nitro,
   9) Benzoyl,
   10) Benzoyl, ein- oder mehrfach substituiert durch
      10.1 Fluor, Chlor, Brom oder Jod,
      10.2 (C₁-C₃)-Alkyl oder
      10.3 (C₁-C₃)-Alkoxy,
   11) Phenoxy oder
   12) Phenoxy, ein oder mehrfach substituiert durch
      12.1 (C₁-C₃)-Alkoxy, ein- oder mehrfach substituiert durch Fluor, Chlor, Brom oder Jod
      12.2 Fluor, Chlor, Brom oder Jod oder
      12.3 (C₁-C₃)-Alkyl, ein- oder mehrfach substituiert durch Fluor, Chlor, Brom oder Jod, steht.

Insbesondere bevorzugt sind die Verbindungen 2-Cyano-3-mercapto-N-(4-trifluormethylphenyl)-crotonsäureamid oder Natrium-2-cyano-N-(4-trifluormethylphenyl)-crotonsäureamid-3-thiolat.

Unter dem Begriff Alkyl oder Alkoxy werden Reste verstanden deren Kohlenstoffkette geradkettig, verzweigt oder cyclisch sein kann. Cyclische Alkylreste sind beispielsweise 3- bis 7-gliedrige Monocyclen wie Cyclopropyl, Cyclobutyl, Cyclopentyl, Cyclohexyl oder Cycloheptyl. Ferner gehört zu den cyclischen Alkylreste auch Polycyclen wie Adamantan-, Twistan- oder Diamantanreste.

Zu dem Begriff "R² und R³ bilden zusammen mit dem Stickstoffatom, an das sie gebunden sind, einen 4- bis 7-gliedrigen Ring" gehören beispielsweise Reste, die sich von Azetidin, Pyrrolidin, Piperidin oder Azepin ableiten.

Zu den "ein-, zwei- oder dreikernigen, ungesättigten heterocyclischen Reste mit 3 bis 13 C-Atomen" gehören beispielsweise Thienyl, Pyridyl, Pyrimidinyl, Pyrazinyl, Pyridazinyl, Imidazolyl, Thiazolyl, Thiazolinyl, Oxazolyl, Thiadiazolyl, Benzoxazolyl, Benzimidazolyl, Chinolyl, Pyrazolyl, Acridinyl, Indolyl, Tetrazolyl oder Indazolyl.

Geeignete physiologisch verträgliche Salze der Verbindung der Formel I sind beispielsweise Alkali-, Erdalkali- und Ammoniumsalze einschließlich solcher von organischen Ammoniumbasen.

Die Erfindung betrifft auch ein Verfahren zur Herstellung der Verbindung der Formel I und/oder ein physiologisch verträgliches Salz der Verbindung der Formel I und/oder eine gegebenenfalls stereoisomere Form der Verbindung der Formel I, das dadurch gekennzeichnet ist, daß man
a) ein Cyanessigsäureamid der Formel V 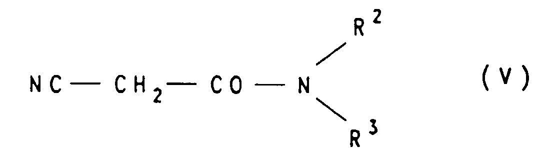 in Gegenwart einer basischen Verbindung mit einer Dithiocarbonsäure der Formel VI

   R¹-CH₂-CS-S-R⁷ (VI)

   umsetzt, wobei R¹, R² und R³, die für die Verbindung der Formel I genannten Bedeutungen haben und R⁷ für C₁-C₆-Alkyl steht, oder
b) eine nach Verfahren a) hergestellte Verbindung der Formel I, die aufgrund ihrer chemischen Struktur in enantiomeren Formen auftritt, durch Salzbildung mit enantiomeren reinen Säuren oder Basen, Chromatographie an chiralen Stationärphasen oder Derivatisierung mittels chiraler enantiomeren reiner Verbindungen wie Aminosäuren, Trennung der somit erhaltenen Diastereomeren, und Abspaltung der chiralen Hilfsgruppe in die reinen Enantiomeren auftrennt, oder
c) die nach Verfahren a) oder b) hergestellte Verbindung der Formel I entweder in freier Form isoliert oder im Falle des Vorliegens von sauren oder basischen Gruppen gegebenenfalls in physiologisch verträgliche Salze umwandelt.

Die Herstellung physiologisch verträglicher Salze aus zur Salzbildung befähigten Verbindungen der Formel I, einschließlich deren stereoisomeren Formen, erfolgt in an sich bekannter Weise. Die Carbonsäuren bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder auch basischen Aminosäuren, etwa Lysin, Ornithin oder Arginin stabile Alkali-, Erdalkali- oder gegebenenfalls substituierte Ammoniumsalze. Sofern die Verbindungen der Formel I basische Gruppen im Rest R³ aufweist, lassen sich mit starken Säuren auch stabile Säureadditionssalze herstellen. Hierfür kommen sowohl anorganische als auch organische Säuren, wie Chlorwasserstoff-, Bromwasserstoff-, Schwefel-, Phosphor-, Methansulfon-, Benzolsulfon-, p-Toluolsulfon-, 4-Brombenzolsulfon-, Cyclohexylamidosulfon-, Trifluormethylsulfon-, Essig-, Oxal-, Wein-, oder Trifluoressigsäure in Frage.

Die Herstellung und Umsetzung der Verbindung der Formel I erfolgt zweckmäßig in einem Verteilungs- oder Lösungsmittel, das sich unter den Reaktionsbedingungen gegenüber den Reaktionspartnern indifferent verhält. Hierfür können beispielsweise Nitrile wie Acetonitril, Ether wie Diethylether, Tetrahydrofuran oder Dioxan und Alkohole wie Methanol, Ethan, Propanol und Isopropanol eingesetzt werden. Als basische Verbindungen können hierbei beispielsweise Alkalihydride, Natrium- oder Kaliumalkoholate von niederen Alkoholen, Alkaliamide, Natrium oder Kalium-Carbonat oder -Bicarbonat und Alkalihydroxide verwendet werden. In einer bevorzugten Ausführungsform wird ein Cyanessigsäureamid der Formel V mit einem Dithiocarbonsäureethylester der Formel VI in Gegenwart eines Natrium- oder Kaliumalkoholats thioacyliert. Als Lösungs- oder Verteilungsmittel wird dabei der zur Herstellung des Alkoholats benutzte Alkohol verwendet. Die als Ausgangsstoffe benötigen Cyanessigsäureamide der Formel V sind bekannt oder lassen sich analog aus Cyanessigsäure und den Aminen der Formel VII 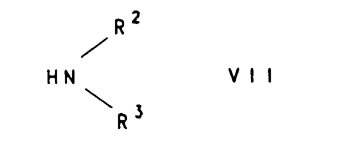 in der R² und R³ die oben angebenene Bedeutungen haben, herstellen. Die Darstellung der Dithiocarbonsäureester der Formel VI erfolgt durch Alkylieren der Dithiocarbonsäuren mit Alkylhalogenamiden oder Dialkylsulfaten.

Die Erfindung betrifft auch Arzneimittel, gekennzeichnet durch einen wirksamen Gehalt an mindestens einer Verbindung der Formel I 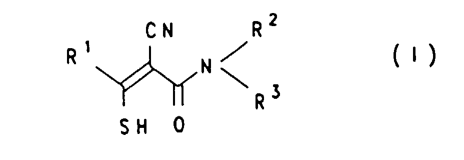 und/oder eines physiologisch verträglichen Salzes der Verbindung der Formel I und/oder eine gegebenenfalls stereoisomere Form der Verbindung der Formel I, wobei die Reste R¹, R² und R³ wie in Formel I definiert sind, zusammen mit einem pharmazeutisch geeigneten und physiologisch verträglichen Trägerstoff, Zusatzstoff und/oder anderen Wirk- und Hilfsstoffen.

Aufgrund ihrer pharmakologischen Eigenschaften eignen sich die erfindungsgemäßen Verbindungen hervorragend zur Behandlung und Prophylaxe von Immun- oder Autoimmunerkrankungen, Erkrankungen mit erhöhtem Zellwachstum wie Krebs oder Restenose, Abstoßungsreaktionen bei Transplantationen, Hauterkrankungen aus der Gruppe Psoriasis, Psoriasis vulgaris, Psoriasis eruptiva, Psoriasis erythrodermic, Psoriasis pustular, Dermatitis, Dermatitis atopica, Dermatitis allergica, Dermatitis photoallergica, Dermatitis medicamentosa und Ekzeme, Asthma, Urticaria, Rhinitis, Uveitis, Typ-II-Diabetes, Leberfibrose, zystische Fibrose, Colitis oder Allergie.

Zu den Immun- oder Autoimmunerkrankungen gehören systemischer Lupus erythematodes, multiple Sklerose, rheumatoide Arthritis, nephrotisches Syndrom, insbesondere Glomerulonephritis, ulzerative Colitis oder juveniler Diabetes.

Zu den Krebserkrankungen gehören Lungenkrebs, Leukämie, Eierstockkrebs, Sarkome, Kaposi's Sarkom, Meningiom, Darmkrebs, Lymphknotenkrebs, Glioblastom, Prostatakrebs oder Hautkrebs.

Bei Organverpflanzungen oder Transplantationen kann es zu Abstoßungsreaktionen des Organ-empfängers gegen das verpflanzte Organ oder zu Abstoßungsreaktionen des verpflanzten Organs gegen den Empfänger kommen ( Host-versus-graft-Reaktion bzw. Graft-versus-host-Reaktion). Unter Abstoßungsreaktionen sind alle Reaktionen des Empfängerorganismus oder des verpflanzten Organs gemeint, die letzlich zum Zell- oder Gewebsuntergang des übertragenen Organs führen bzw. die Funktions- und Lebensfähigkeit des übertragenen Organs oder den Empfänger beeinträchtigen. Insbesondere sind akute wie auch chronische Abstoßungsreaktionen gemeint.

Unter dem Begriff Organ werden alle Organe bzw. Organteile (auch mehrere) bei Säugetieren, insbesondere des Menschen, verstanden, beispielsweise Niere, Herz, Haut, Leber, Muskel, Hornhaut, Knochen, Knochenmark, Lunge, Bauchspeicheldrüse, Darm oder Magen.

Die Erfindung betrifft ferner die Verwendung der Verbindung der Formel I zur Herstellung von Arzneimitteln zur Prophylaxe und Therapie von Immun- oder Autoimmunerkrankungen, Krankheiten mit erhöhtem Zellwachstum wie Krebs oder Restenose, Abstoßungsreaktionen bei Transplantationen, Hauterkrankungen aus der Gruppe Psoriasis, Psoriasis vulgaris, Psoriasis eruptiva, Psoriasis erythrodermic, Psoriasis pustular, Dermatitis, Dermatitis atopica, Dermatitis allergica, Dermatitis photoallergica, Dermatitis medicamentosa und Ekzeme, Asthma, Urticaria, Rhinitis, Uveitis, Typ-II-Diabetes, Leberfibrose, zystische Fibrose, Colitis oder Allergie.

Die Erfindung betrifft auch ein Verfahren zur Herstellung eines Arzneimittels, das dadurch gekennzeichnet ist, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind - je nach Wirksamkeit der Verbindungen gemäß Formel I, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

### Beispiel 1

### 2-Cyano-3-mercapto-N-(4-trifluormethylphenyl)crotonsäureamid

a) N-(4-Trifluormethylphenyl)cyanessigsäureamid
   Die Herstellung erfolgt analog zu GB 930 808. In eine Lösung von Cyanessigsäure (34,0 g; 0,40 Mol) in 1,2 1 Dichlormethan werden Phosphorpentachlorid (83,3 g; 0,40 Mol) eingetragen und 30 Minuten unter Rückfluß erhitzt. Man gibt innerhalb von etwa 10 Minuten das 4-Trifluormethylanilin (41 g; 0,26 Mol) in die Lösung und erhitzt für weitere 2 Stunden am Rückfluß. Nach Abkühlen auf 20°C wird das Reaktionsgemisch mit 500 ml Wasser versetzt und für 30 Minuten nachgerührt. Die wässrige Phase wird mit Natriumcarbonat neutralisiert und der Niederschlag wird abgenutscht. Der Filterkuchen wird mit Wasser gewaschen und getrocknet. Man erhält so 55,2 g N-(4-Trifluormethylphenyl)cyanessigsäureamid in kristalliner Form. Schmelzpunkt: 194-195,5°C.
b) N-(4-Trifluormethylphenyl)cyanessigsäureamid (9,1 g; 0,04 Mol) werden in Ethanol (150 ml) suspendiert und bei 0°C bis 5°C mit Kaliumtertiärbutylat (4,8 g; 0,04 Mol) versetzt. Anschließend wird Dithioessigsäureethylester (4,9 g; 0,04 Mol) unter Rühren bei 15°C bis 20°C zugetropft. Die Reaktionsmischung wird auf 70°C bis 75°C erhitzt. Nach beendeter Mercaptanentwicklung rührt man noch 30 Minuten bei 65°C bis 70°C. Nach Abkühlen auf Raumtemperatur werden 100 ml Wasser in das Reaktionsgemisch getropft und man läßt 15 Minuten bei Raumtemperatur nachrühren. Das Reaktionsgemisch wird auf 400 ml 0,1 N Salzsäure gegossen und 4 mal mit je 100 ml Dichlormethan ausgeschüttelt. Die vereinigten organischen Phasen werden 2 mal mit je 300 ml Wasser gewaschen, über Natriumsulfat getrocknet und unter verminderten Druck bis zur Trockene eingeengt. Der Rückstand (10,5 g) wird aus 260 ml Ethanol kristallisiert. Man erhält so 5,6 g 2-Cyano-3-mercapto-N-(4-trifluormethylphenyl)crotonsäureamid als gelbliche Kristalle. Schmelzpunkt: 183°C bis 184°C

### Beispiel 2

### Natrium-2-cyano-N-(4-trifluormethylphenyl)crotonsäureamid-3-thiolat:

2-Cyano-3-mercapto-N-(4-trifluormethylphenyl)crotonsäureamid (2,6 g; 0,09 Mol) aus Beispiel 1 werden in Wasser (450 ml) suspendiert und tropfenweise unter Rühren mit einer Lösung von Natriumhydroxyd (0,35 g; 0,009 Mol) in Wasser (10 ml) bis 45°C versetzt bis die Reaktionsmischung einen pH-Wert von 5,4 besitzt. Es wird filtriert und das Filtrat wird unter verminderten Druck bei 50°C bis zur Trockene eingeengt. Man erhält so 3,0 g Natrium-2-cyano-N-(4-trifluormethylphenyl)crotonsäureamid-3-thiolat als blaßgelbe Kristalle.
Schmelzpunkt: 235°C bis 240°C (Zersetzung)

### Pharmakologische Prüfungen

### 1. Adjuvans induzierte Arthritis, Modifikation nach Perper (Proc. Soc. exp. Biol. Med. 137, 506 (1971)

Als Versuchstiere dienen männliche Ratten eines Wistar-Lewis-Stammes mit einem Körpergewicht von 130 bis 200 g. Die Tiere erhalten am 1. Tag eine subkutane Injektion in den Schwanz von 0,1 ml einer Mycobacterium butyricum Suspension (Difco; 6 mg/kg in Paraffin Öl; Merck). Die zu vergleichenden Verbindungen werden zweimal täglich vom 1. bis zum 12. Versuchstag intraperitoneal appliziert; dann erfolgt die Bestimmung als Pfotenvolumens am 18. Tag.

Tiere einer Kontrollgruppe erhalten nur das Lösungsmittel. Pro Dosierung und in der Kontrollgruppe werden jeweils 6 Tiere verwendet. Als Wirkungskriterium dient die Herabsetzung der Pfotenvolumenzunahme gegenüber der unbehandelten Kontrollgruppe.

Eine Dosis von 25 mg der Verbindung gemäß Beispiel 2 pro kg Lebendgewicht einer Wistar-Lewis Ratte ergibt eine 83 % Hemmung (38 % Hemmung bei 12,6 mg/kg) der Pfotenvolumenzunahme im Vergleich mit einer unbehandelten Kontrollgruppe.

## Patentansprüche

1. Verbindung der Formel I, 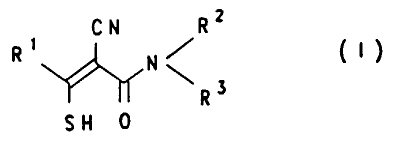 und/oder eine gegebenenfalls stereoisomere Form der Verbindung der Formel I und/oder ein physiologisch verträgliches Salz der Verbindung der Formel I, wobei
R¹ für
a) Wasserstoffatom,
b) (C₁-C₁₇)-Alkyl,
c) (C₁-C₄)-Alkyl, ein- oder mehrfach substituiert durch Fluor, Chlor, Brom oder Jod,
d) Phenyl,
e) Phenyl, ein- oder mehrfach substituiert durch
1) Fluor, Chlor, Brom oder Jod,
2) Nitro,
3) Cyano,
4) (C₁-C₄)-Alkyl,
5) (C₁-C₄)-Alkyl, ein- oder mehrfach substituiert durch Fluor, Chlor, Brom oder Jod,
6) (C₁-C₄)-Alkoxy,
7) (C₁-C₄)-Alkoxy, ein- oder mehrfach substituiert durch Fluor, Chlor, Brom oder Jod,Phenyl-(C₁-C₂)-alkyl,
f) Benzyl,
g) (C₃-C₇)-Cycloalkyl,
h) Alkenyl mit 2 oder 3 C-Atomen oder
i) Alkinyl mit 2 oder 3 C-Atomen, steht,
R² für
a) Wasserstoffatom,
b) (C₁-C₄)-Alkyl,
c) Phenyl,
d) Phenyl-(C₁-C₂)-alkyl oder
e) Alkenyl mit 2 bis 3 C-Atomen, steht, oder
R³ für
a) einen ein-, zwei- oder dreikernigen, ungesättigten heterocyclischen Rest mit 3 bis 13 C-Atomen und 1 bis 4 Heteroatomen aus der Gruppe Sauerstoff, Schwefel und Stickstoff steht, wovon im Ringsystem höchstens eines der Heteroatome von Stickstoff verschieden ist, und unsubstituiert ist oder ein- oder mehrfach substituiert ist durch
1) Fluor, Chlor, Brom oder Jod,
2) (C₁-C₄)-Alkyl,
3) (C₁-C₄)-Alkyl, ein- oder mehrfach substituiert durch Fluor, Chlor, Brom oder Jod,
4) (C₁-C₄)-Alkoxy,
5) (C₁-C₄)-Alkoxy, ein- oder mehrfach substituiert durch Fluor, Chlor, Brom oder Jod
6) Nitro,
7) Hydroxy,
8) Carboxy,
9) Carbamoyl oder
10) Oxogruppe,
b) einen Rest der Formel II, 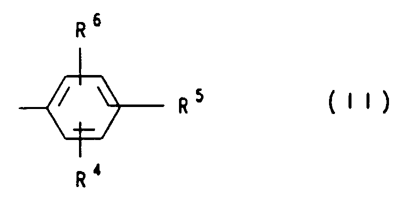 in der R⁴, R⁵ und R⁶ gleich oder verschieden sein können und für
1) Wasserstoffatom,
2) (C₁-C₄)-Alkyl,
3) (C₁-C₄)-Alkyl, ein- oder mehrfach substituiert durch Fluor, Chlor, Brom oder Jod,
4) in der R⁴ für Wasserstoffatom und R⁵ und R⁶ gemeinsam mit dem Phenylring der Formel II einen Naphthalinring bilden,
5) in der R⁴ für Wasserstoffatom und R⁵ und R⁶ einen Methylendioxyrest bilden,
6) (C₁-C₄)-Alkoxy,
7) (C₁-C₄)-Alkoxy, ein- oder mehrfach substituiert durch Fluor, Chlor, Brom oder Jod,
8) (C₁-C₄)-Alkylmercapto,
9) (C₁-C₄)-Alkylmercapto, ein- oder mehrfach substituiert durch Fluor, Chlor, Brom oder Jod,
10) Fluor, Chlor, Brom oder Jod,
11) Nitro,
12) Cyano,
13) Hydroxy,
14) Carboxy,
15) (C₁-C₄)-Alkylsulfonyl,
16) Carbalkoxy, mit 1 bis 3 C-Atomen in der Alkylkette,
17) Benzoyl,
18) Benzoyl, ein- oder mehrfach substituiert durch
18.1 Fluor, Chlor, Brom oder Jod,
18.2 (C₁-C₄)-Alkyl oder
18.3 (C₁-C₄)-Alkoxy,
19) Phenyl,
20) Phenyl, ein- oder mehrfach substituiert durch
20.1 (C₁-C₄)-Alkoxy,
20.2 Fluor, Chlor, Brom oder Jod oder
20.3 (C₁-C₄)-Alkyl,
21) Phenoxy, oder
22) Phenoxy, ein- oder mehrfach substituiert durch (C₁-C₃)-Alkoxy, ein- oder mehrfach substituiert durch
22.1 Fluor, Chlor, Brom oder Jod,
22.2 (C₁-C₃)-Alkyl, ein- oder mehrfach substituiert durch Fluor, Chlor, Brom oder Jod, oder
22.3 Fluor, Chlor, Brom oder Jod,
c) einen Rest der Formel III steht,
-(CH₂)ₙ-COOR¹⁰ (III)
in der R¹⁰ für
1) Wasserstoffatom oder
2) (C₁-C₄)-Alkyl und
n für eine ganze Zahl von 1 bis 12 steht, oder
d) R² und R³ bilden zusammen mit dem Stickstoffatom, an das sie gebunden sind, einen 4- bis 7-gliedrigen Ring, der unsubstituiert oder substituiert ist durch Carbonyl am N-Atom benachbarten C-Atom, oder
e) R² und R³ bilden zusammen mit dem Stickstoffatom, an das sie gebunden sind, einen 5- bis 6-gliedrigen Ring der Formel IV 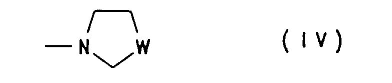 in der W für
1)
-CH₂-,
2)
-CH₂-CH₂-,
3) 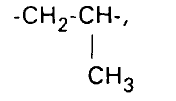
4) 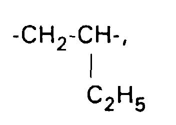
5) 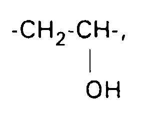
6)
-CH₂-O-
oder
7)
-CH₂-S-
steht.

2. Verbindung der Formel I gemäß Anspruch 1 und/oder ein physiologisch verträgliches Salz der Verbindung der Formel und/oder eine gegebenenfalls stereoisomere Form der Verbindung der Formel I, wobei
R¹ für
a) Wasserstoffatom,
b) (C₁-C₆)-Alkyl,
c) (C₁-C₄)-Alkyl, ein- oder mehrfach substituiert durch Fluor, Chlor, Brom oder Jod,
d) Phenyl,
e) Alkenyl mit 2 oder 3 C-Atomen oder
f) (C₃-C₄)-Cyloalkyl, steht
R² für
a) Wasserstoffatom,
b) (C₁-C₄)-Alkyl,
c) Benzyl oder
d) Alkenyl mit 2 bis 3 C-Atomen steht,
R³ für
a) Pyridyl ein- oder mehrfach substituiert durch
1) Wasserstoffatom,
2) Fluor, Chlor, Brom oder Jod,
3) Nitro,
4) (C₁-C₃)-Alkyl oder
5) (C₁-C₃)-Alkoxy,
b) einen Rest der Formel II, in der R⁴, R⁵ und R⁶ gleich oder verschieden sein können und für
1) Wasserstoffatom,
2) (C₁-C₃)-Alkyl,
3) (C₁-C₃)-Alkyl, ein oder mehrfach substituiert durch Fluor, Chlor, Brom oder Jod,
4) in der R⁴ für Wasserstoffatom steht und R⁵ und R⁶ einen Methylendioxyrest bilden,
5) (C₁-C₃)-Alkoxy,
6) (C₁-C₃)-Alkoxy, ein- oder mehrfach substituiert durch Fluor, Chlor, Brom oder Jod,
7) (C₁-C₃)-Alkylmercapto,
8) (C₁-C₃)-Alkylmercapto, ein- oder mehrfach substituiert durch Fluor, Chlor, Brom oder Jod,
9) Fluor, Chlor, Brom oder Jod,
10) Nitro,
11) Cyano,
12) (C₁-C₃)-Alkylsulfonyl,
13) Benzoyl,
14) Benzoyl, ein- oder mehrfach substituiert durch
14.1 Fluor, Chlor, Brom oder Jod
14.2 (C₁-C₃)-Alkyl oder
14.3 (C₁-C₃)-Alkoxy,
15) Phenoxy, oder
16) Phenoxy, ein- oder mehrfach substituiert durch
16.1 (C₁-C₃)-Alkoxy, ein- oder mehrfach substituiert durch Fluor, Chlor, Brom oder Jod,
16.2 Fluor, Chlor, Brom oder Jod,
16.3 (C₁-C₃)-Alkyl, ein- oder mehrfach substituiert durch Fluor, Chlor, Brom, oder Jod
c) einen Rest der Formel III steht und in der R¹⁰ für
1) Wasserstoffatom,
2) (C₁-C₄)-Alkyl und
n für eine ganze Zahl von 1 bis 12 steht, oder
d) R² und R³ bilden zusammen mit dem Stickstoffatom, an das sie gebunden sind, einen 4- bis 7-gliedrigen Ring, der durch Carbonyl am N-Atom benachbarten C-Atom substituiert ist.

3. Verbindung der Formel I gemäß der Ansprüche 1 oder 2, wobei
R¹ für (C₁-C₆)-Alkyl steht,
R² für Wasserstoffatom steht,
R³ für
a) Pyridyl, ein- oder mehrfach substituiert durch Fluor, Chlor, Brom oder Jod, oder
b) einen Rest der Formel II, in der R⁴, R⁵ und R⁶ gleich oder verschieden sein können und für
1) Wasserstoffatom oder,
2) (C₁-C₃)-Alkyl,
3) (C₁-C₃)-Alkyl ein- oder mehrfach substituiert durch Fluor, Chlor, Brom oder Jod,
4) in der R⁴ für Wasserstoffatom und R⁵ und R⁶ einen Methylendioxyrest bilden,
5) (C₁-C₃)-Alkoxy,
6) (C₁-C₃)-Alkoxy, ein- oder mehrfach substituiert durch Fluor, Chlor, Brom oder Jod,
7) Fluor, Chlor, Brom oder Jod,
8) Nitro,
9) Benzoyl,
10) Benzoyl, ein- oder mehrfach substituiert durch
10.1 Fluor, Chlor, Brom oder Jod,
10.2 (C₁-C₃)-Alkyl oder
10.3 (C₁-C₃)-Alkoxy,
11) Phenoxy oder
12) Phenoxy, ein oder mehrfach substituiert durch
12.1 (C₁-C₃)-Alkoxy, ein- oder mehrfach substituiert durch Fluor, Chlor, Brom oder Jod
12.2 Fluor, Chlor, Brom oder Jod oder
12.3 (C₁-C₃)-Alkyl, ein- oder mehrfach substituiert durch Fluor, Chlor, Brom oder Jod, steht.

4. 2-Cyano-3-mercapto-N-(4-trifluormethylphenyl)-crotonsäureamid oder Natrium-2-cyano-N-(4-trifluormethylphenyl)-crotonsäureamid-3-thiolat.

5. Verfahren zur Herstellung der Verbindung der Formel I, wobei man
a) ein Cyanessigsäureamid der Formel V 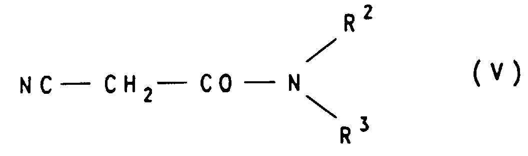 in Gegenwart einer basischen Verbindung mit einer Dithiocarbonsäure der Formel VI
R¹-CH₂-CS-S-R⁷ (VI)
umsetzt, wobei R¹, R² und R³, die für die Verbindung der Formel I genannten Bedeutungen haben und R⁷ für C₁-C₆-Alkyl steht, oder
b) eine nach Verfahren a) hergestellte Verbindung der Formel I, die aufgrund ihrer chemischen Struktur in enantiomeren Formen auftritt, durch Salzbildung mit enantiomeren reinen Säuren oder Basen, Chromatographie an chiralen Stationärphasen oder Derivatisierung mittels chiraler enantiomeren reiner Verbindungen wie Aminosäuren, Trennung der somit erhaltenen Diastereomeren, und Abspaltung der chiralen Hilfsgruppe in die reinen Enantiomeren auftrennt, oder
c) die nach Verfahren a) oder b) hergestellte Verbindung der Formel I entweder in freier Form isoliert oder im Falle des Vorliegens von sauren oder basischen Gruppen gegebenenfalls in physiologisch verträgliche Salze umwandelt.

6. Arzneimittel, enthaltend eine wirksame Menge mindestens einer Verbindung der Formel I gemäß Anspruch 1 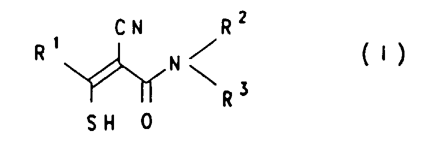 und/oder eines physiologisch verträglichen Salzes der Verbindung der Formel I und/oder eine gegebenenfalls stereoisomere Form der Verbindung der Formel I, wobei die Reste R¹, R² und R³ wie in Anspruch 1 definiert sind, zusammen mit einem pharmazeutisch geeigneten und physiologisch verträglichen Trägerstoff, Zusatzstoff und/oder anderen Wirk- und Hilfsstoffen.

7. Verwendung von mindestens einer Verbindung der Formel I nach einem oder mehreren der Ansprüche 1 bis 4, zur Herstellung von Arzneimitteln zur Behandlung und Prophylaxe von Immun- oder Autoimmunerkrankungen, Erkrankungen mit erhöhtem Zellwachstum wie Krebs oder Restenose, Abstoßungsreaktionen bei Transplantationen, Hauterkrankungen aus der Gruppe Psoriasis, Psoriasis vulgaris, Psoriasis eruptiva, Psoriasis erythrodermic, Psoriasis pustular, Dermatitis, Dermatitis atopica, Dermatitis allergica, Dermatitis photoallergica, Dermatitis medicamentosa und Ekzeme, Asthma, Urticaria, Rhinitis, Uveitis, Typ-II-Diabetes, Leberfibrose, zystische Fibrose, Colitis oder Allergie.

8. Verfahren zur Herstellung eines Arzneimittels nach Anspruch 6, dadurch gekennzeichnet, daß man mindestens eine Verbindung der Formel I gemäß Anspruch 1 und/oder eine nach dem Verfahren gemäß Anspruch 5 erhaltene Verbindung mit physiologisch annehmbaren Hilfs- und Trägerstoffen und gegebenenfalls weiteren Zusatzstoffen und/oder anderen Wirkstoffen in eine geeignete Darreichungsform bringt.

## Claims

1. A compound of the formula I 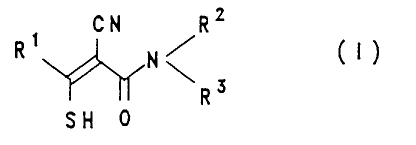 and/or an optionally stereoisomeric form of the compound of the formula I and/or a physiologically tolerable salt of the compound of the formula I, where
R¹ is
a) a hydrogen atom,
b) (C₁-C₁₇)-alkyl,
c) (C₁-C₄)-alkyl, mono- or polysubstituted by fluorine, chlorine, bromine or iodine,
d) phenyl,
e) phenyl, mono- or polysubstituted by
1) fluorine, chlorine, bromine or iodine,
2) nitro,
3) cyano,
4) (C₁-C₄)-alkyl,
5) (C₁-C₄)-alkyl, mono- or polysubstituted by fluorine, chlorine, bromine or iodine,
6) (C₁-C₄)-alkoxy,
7) (C₁-C₄)-alkoxy, mono- or polysubstituted by fluorine, chlorine, bromine or iodine, phenyl-(C₁-C₂)-alkyl,
f) benzyl,
g) (C₃-C₇)-cycloalkyl,
h) alkenyl having 2 or 3 carbon atoms or
i) alkynyl having 2 or 3 carbon atoms,
R² is
a) a hydrogen atom,
b) (C₁-C₄)-alkyl,
c) phenyl,
d) phenyl-(C₁-C₂)-alkyl or
e) alkenyl having 2 to 3 carbon atoms, or
R³ is
a) a mono-, di- or trinuclear, unsaturated heterocyclic radical having 3 to 13 carbon atoms and 1 to 4 heteroatoms from the group consisting of oxygen, sulfur and nitrogen, of which at most one of the heteroatoms in the ring system is other than nitrogen, and is unsubstituted or mono- or polysubstituted by
1) fluorine, chlorine, bromine or iodine,
2) (C₁-C₄)-alkyl,
3) (C₁-C₄)-alkyl, mono- or polysubstituted by fluorine, chlorine, bromine or iodine,
4) (C₁-C₄)-alkoxy,
5) (C₁-C₄)-alkoxy, mono- or polysubstituted by fluorine, chlorine, bromine or iodine,
6) nitro,
7) hydroxyl,
8) carboxyl,
9) carbamoyl or
10) an oxo group,
b) a radical of the formula II 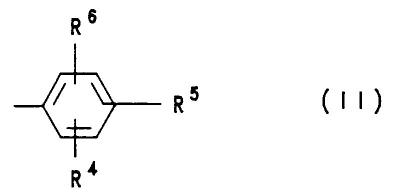 in which R⁴, R⁵ and R⁶ can be identical or different and are
1) a hydrogen atom,
2) (C₁-C₄)-alkyl,
3) (C₁-C₄)-alkyl, mono- or polysubstituted by fluorine, chlorine, bromine or iodine,
4) in which R⁴ is a hydrogen atom and R⁵ and R⁶, together with the phenyl ring of the formula II, form a naphthalene ring,
5) in which R⁴ is a hydrogen atom and R⁵ and R⁶ form a methylenedioxy radical,
6) (C₁-C₄)-alkoxy,
7) (C₁-C₄)-alkoxy, mono- or polysubstituted by fluorine, chlorine, bromine or iodine,
8) (C₁-C₄)-alkylmercapto,
9) (C₁-C4)-alkylmercapto, mono- or polysubstituted by fluorine, chlorine, bromine or iodine,
10) fluorine, chlorine, bromine or iodine,
11) nitro,
12) cyano,
13) hydroxyl,
14) carboxyl,
15) (C₁-C₄)-alkylsulfonyl,
16) carbalkoxy, having 1 to 3 carbon atoms in the alkyl chain,
17) benzoyl,
18) benzoyl, mono- or polysubstituted by
18.1 fluorine, chlorine, bromine or iodine,
18.2 (C₁-C₄)-alkyl or
18.3 (C₁-C₄)-alkoxy,
19) phenyl,
20) phenyl, mono- or polysubstituted by
20.1 (C₁-C₄)-alkoxy,
20.2 fluorine, chlorine, bromine or iodine or
20.3 (C₁-C₄)-alkyl,
21) phenoxy, or
22) phenoxy, mono- or polysubstituted by (C₁-C₃)-alkoxy, mono- or polysubstituted by
22.1 fluorine, chlorine, bromine or iodine,
22.2 (C₁-C₃)-alkyl, mono- or polysubstituted by fluorine, chlorine, bromine or iodine, or
22.3 fluorine, chlorine, bromine or iodine,
c) a radical of the formula III
-(CH₂)ₙ-COOR¹⁰ (III)
in which R¹⁰ is
1) a hydrogen atom or
2) (C₁-C₄)-alkyl and
n is an integer from 1 to 12, or
d) R² and R³, together with the nitrogen atom to which they are bonded, form a 4- to 7-membered ring, which is unsubstituted or substituted by carbonyl on the carbon atom adjacent to the nitrogen atom, or
e) R² and R³, together with the nitrogen atom to which they are bonded, form a 5- to 6-membered ring of the formula IV 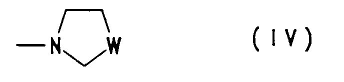 in which W is
1)
-CH₂-,
2)
-CH₂-CH₂-,
3) 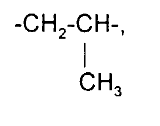
4) 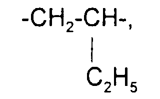
5) 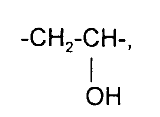
6)
-CH₂-O-
or
7)
-CH₂-S-.

2. A compound of the formula I as claimed in claim 1 and/or a physiologically tolerable salt of the compound of the formula I and/or an optionally stereoisomeric form of the compound of the formula I, where
R¹ is
a) a hydrogen atom,
b) (C₁-C₆)-alkyl,
c) (C₁-C₄)-alkyl, mono- or polysubstituted by fluorine, chlorine, bromine or iodine,
d) phenyl,
e) alkenyl having 2 or 3 carbon atoms or
f) (C₃-C₄)-cycloalkyl,
R² is
a) a hydrogen atom,
b) (C₁-C₄)-alkyl,
c) benzyl or
d) alkenyl having 2 to 3 carbon atoms,
R³ is
a) pyridyl, mono- or polysubstituted by
1) a hydrogen atom,
2) fluorine, chlorine, bromine or iodine,
3) nitro,
4) (C₁-C₃)-alkyl or
5) (C₁-C₃)-alkoxy,
b) a radical of the formula II in which R⁴, R⁵ and R⁶ can be identical or different and are
1) a hydrogen atom,
2) (C₁-C₃)-alkyl,
3) (C₁-C₃)-alkyl, mono- or polysubstituted by fluorine, chlorine, bromine or iodine,
4) in which R⁴ is a hydrogen atom and R⁵ and R⁶ form a methylenedioxy radical,
5) (C₁-C₃)-alkoxy,
6) (C₁-C₃)-alkoxy, mono- or polysubstituted by fluorine, chlorine, bromine or iodine,
7) (C₁-C₃)-alkylmercapto,
8) (C₁-C₃)-alkylmercapto, mono- or polysubstituted by fluorine, chlorine, bromine or iodine,
9) fluorine, chlorine, bromine or iodine,
10) nitro,
11) cyano,
12) (C₁-C₃)-alkylsulfonyl,
13) benzoyl,
14) benzoyl, mono- or polysubstituted by
14.1 fluorine, chlorine, bromine or iodine,
14.2 (C₁-C₃)-alkyl or
14.3 (C₁-C₃)-alkoxy,
15) phenoxy, or
16) phenoxy, mono- or polysubstituted by
16.1 (C₁-C₃)-alkoxy, mono- or polysubstituted by fluorine, chlorine, bromine or iodine,
16.2 fluorine, chlorine, bromine or iodine,
16.3 (C₁-C₃)-alkyl, mono- or polysubstituted by fluorine, chlorine, bromine or iodine,
c) a radical of the formula III in which R¹⁰ is
1) a hydrogen atom,
2) (C₁-C₄)-alkyl and
n is an integer from 1 to 12, or
d) R² and R³, together with the nitrogen atom to which they are bonded, form a 4- to 7-membered ring which is substituted by carbonyl on the carbon atom adjacent to the nitrogen atom.

3. A compound of the formula I as claimed in claims 1 and 2, where
R¹ is (C₁-C₆)-alkyl,
R² is a hydrogen atom,
R³ is
a) pyridyl, mono- or polysubstituted by fluorine, chlorine, bromine or iodine, or
b) a radical of the formula II in which R⁴, R⁵ and R⁶ can be identical or different and are
1) a hydrogen atom or
2) (C₁-C₃)-alkyl,
3) (C₁-C₃)-alkyl, mono- or polysubstituted by fluorine, chlorine, bromine or iodine,
4) in which R⁴ is a hydrogen atom and R⁵ and R⁶ form a methylenedioxy radical,
5) (C₁-C₃)-alkoxy,
6) (C₁-C₃)-alkoxy, mono- or polysubstituted by fluorine, chlorine, bromine or iodine,
7) fluorine, chlorine, bromine or iodine,
8) nitro,
9) benzoyl,
10) benzoyl, mono- or polysubstituted by
10.1 fluorine, chlorine, bromine or iodine,
10.2 (C₁-C₃)-alkyl or
10.3 (C₁-C₃)-alkoxy,
11) phenoxy or
12) phenoxy, mono- or polysubstituted by
12.1 (C₁-C₃)-alkoxy, mono- or polysubstituted by fluorine, chlorine, bromine or iodine,
12.2 fluorine, chlorine, bromine or iodine or
12.3 (C₁-C₃)-alkyl, mono- or polysubstituted by fluorine, chlorine, bromine or iodine.

4. 2-Cyano-3-mercapto-N-(4-trifluoromethylphenyl)crotonamide or sodium 2-cyano-N-(4-trifluoromethylphenyl)crotonamide-3-thiolate.

5. A process for the preparation of the compound of the formula I, where
a) a cyanoacetamide of the formula V 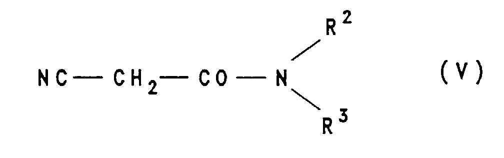 is reacted in the presence of a basic compound with a dithiocarboxylic acid of the formula VI
R¹-CH₂-CS-S-R⁷ (VI)
where R¹, R² and R³ have the meanings mentioned for the compound of the formula I and R⁷ is C₁-C₆-alkyl, or
b) a compound of the formula I prepared according to process a), which on account of its chemical structure occurs in enantiomeric forms, is resolved into the pure enantiomers by salt formation with enantiomerically pure acids or bases, chromatography on chiral stationary phases or derivatization by means of chiral enantiomerically pure compounds such as amino acids, separation of the diastereomers thus obtained, and removal of the chiral auxiliary group, or
c) the compound of the formula I prepared according to process a) or b) is either isolated in free form or, in the case of the presence of acidic or basic groups, optionally converted into physiologically tolerable salts.

6. A pharmaceutical, comprising an efficacious amount of at least one compound of the formula I as claimed in claim 1 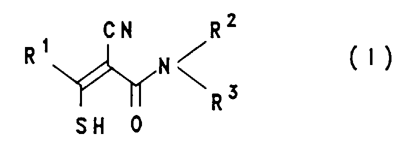 and/or of a physiologically tolerable salt of the compound of the formula I and/or an optionally stereoisomeric form of the compound of the formula I, where the radicals R¹, R² and R³ are as defined in claim 1, together with a pharmaceutically suitable and physiologically tolerable excipient, additive and/or other active compounds and auxiliaries.

7. The use of at least one compound of the formula I as claimed in one or more of claims 1 to 4 for the production of pharmaceuticals for the treatment and prophylaxis of immune or autoimmune disorders, disorders with increased cell growth such as cancer or restenosis, rejection reactions in transplants, skin disorders from the group consisting of psoriasis, psoriasis vulgaris, psoriasis eruptiva, erythrodermic psoriasis, pustular psoriasis, dermatitis, atopic dermatitis, allergic dermatitis, photoallergic dermatitis, dermatitis medicamentosa and eczema, asthma, urticaria, rhinitis, uveitis, type II diabetes, liver fibrosis, cystic fibrosis, colitis or allergy.

8. A process for the production of a pharmaceutical as claimed in claim 6, which comprises bringing at least one compound of the formula I as claimed in claim 1 and/or a compound obtained by the process as claimed in claim 5 into a suitable administration form with physiologically acceptable auxiliaries and excipients and, if appropriate, further additives and/or other active compounds.

## Revendications

1. Composé de formule I 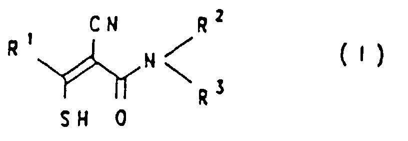 et/ou forme éventuellement stéréoisomère du composé de formule I et/ou sel physiologiquement acceptable du composé de formule I,
formule dans laquelle
R¹ représente
a) un atome d'hydrogène,
b) un groupe alkyle en C₁-C₁₇,
c) un groupe alkyle en C₁-C₄, une ou plusieurs fois substitué par l'atome de fluor, chlore, brome ou iode,
d) le groupe phényle,
e) un groupe phényle une ou plusieurs fois substitué par
1) un atome de fluor, chlore, brome ou iode,
2) le groupe nitro,
3) le groupe cyano,
4) un groupe alkyle en C₁-C₄,
5) un groupe alkyle en C₁-C₄ une ou plusieurs fois substitué par l'atome de fluor, chlore, brome ou iode,
6) un groupe alcoxy en C₁-C₄,
7) un groupe alcoxy en C₁-C₄ une ou plusieurs fois substitué par l'atome de fluor, chlore, brome ou iode,
f) le groupe benzyle,
g) un groupe cycloalkyle en C₃-C₇,
h) un groupe alcényle à 2 ou 3 atomes de carbone, ou
i) un groupe alcynyle à 2 ou 3 atomes de carbone,
R² représente
a) un atome d'hydrogène,
b) un groupe alkyle en C₁-C₄,
c) le groupe phényle,
d) un groupe phényl-alkyle(C₁-C₂), et
e) un groupe alcényle à 2 ou 3 atomes de carbone, et
R³ représente
a) un radical hétérocyclique insaturé mono-, di- ou trinucléaire, ayant de 3 à 13 atomes de carbone et de 1 à 4 hétéroatomes choisis parmi les atomes d'oxygène, d'azote et de soufre, dont au maximum l'un des hétéroatomes dans le système cyclique est différent d'un atome d'azote, et étant non substitué ou une ou plusieurs fois substitué par
1) l'atome de fluor, chlore, brome ou iode,
2) un groupe alkyle en C₁-C₄,
3) un groupe alkyle en C₁-C₄ une ou plusieurs fois substitué par l'atome de fluor, chlore; brome ou iode,
4) un groupe alcoxy en C₁-C₄,
5) un groupe alcoxy en C₁-C₄ une ou plusieurs fois substitué par l'atome de fluor, chlore, brome ou iode,
6) le groupe nitro,
7) le groupe hydroxyle,
8) le groupe carboxyle,
9) le groupe carbamoyle ou
10) le groupe oxo,
b) un radical de formule II 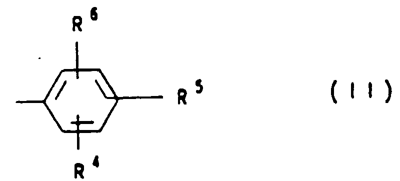 dans laquelle R⁴, R⁵ et R⁶ peuvent être identiques ou différents et représentent
1) un atome d'hydrogène,
2) un groupe alkyle en C₁-C₄,
3) un groupe alkyle en C₁-C₄ une ou plusieurs fois substitué par l'atome de fluor, chlore, brome ou iode,
4) dans laquelle R⁴ représente un atome d'hydrogène et R⁵ et R⁶ forment ensemble, avec le cycle phényle de formule II, un cycle naphtalène,
5) dans laquelle R⁴ représente un atome d'hydrogène et R⁵ et R⁶ forment un radical méthylènedioxy,
6) un groupe alcoxy en C₁-C₄,
7) un groupe alcoxy en C₁-C₄ une ou plusieurs fois substitué par l'atome de fluor, chlore, brome ou iode,
8) un groupe alkyl(C₁-C₄)mercapto,
9) un groupe alkyl(C₁-C₄)mercapto une ou plusieurs fois substitué par l'atome de fluor, chlore, brome ou iode,
10) l'atome de fluore, chlore, brome ou iode,
11) le groupe nitro,
12) le groupe cyano,
13) le groupe hydroxyle,
14) le groupe carboxyle,
15) un groupe alkyl(C₁-C₄)sulfonyle,
16) un groupe carbalcoxy ayant de 1 à 3 atomes de carbone dans la chaîne alkyle,
17) le groupe benzoyle,
18) un groupe benzoyle une ou plusieurs fois substitué par
18.1. l'atome de fluor, chlore, brome ou iode,
18.2. un groupe alkyle en C₁-C₄ ou
18.3. un groupe alcoxy en C₁-C₄,
19) le groupe phényle,
20) un groupe phényle une ou plusieurs fois substitué par
20.1. un groupe alcoxy en C₁-C₄,
20.2. l'atome de fluor, chlore, brome ou iode, ou
20.3. un groupe alkyle en C₁-C₄,
21) le groupe phénoxy ou
22) un groupe phénoxy une ou plusieurs fois substitué par un groupe alcoxy en C₁-C₃ une ou plusieurs fois substitué par
22.1. l'atome de fluor, chlore, brome ou iode,
22.2. un groupe alkyle en C₁-C₃ une ou plusieurs fois substitué par l'atome de fluor, chlore, brome ou iode, ou
22.3. l'atome de fluor, chlore, brome ou iode,
c) un radical de formule III
-(CH₂)ₙ-COOR¹⁰ (III)
dans laquelle
R¹⁰ représente
1) un atome d'hydrogène ou
2) un groupe alkyle en C₁-C₄, et
n représente un nombre entier allant de 1 à 12, ou
d) R² et R³ forment ensemble, avec l'atome d'azote auquel ils sont liés, un cycle à 4-7 chaînons, qui n'est pas substitué ou est substitué par le groupe carbonyle sur l'atome de carbone contigu à l'atome d'azote, ou
e) R² et R³ forment ensemble, avec l'atome d'azote auquel ils sont liés, un cycle à 5-6 chaînons de formule IV 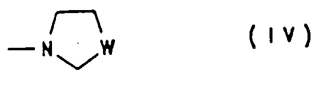 dans laquelle W représente
1)
-CH₂-,
2)
-CH₂-CH₂-,
3) 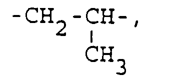
4) 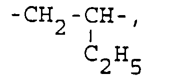
5) 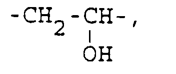
6)
-CH₂-O-
ou
7)
-CH₂-S-.

2. Composé de formule I selon la revendication 1 et/ou sel physiologiquement acceptable du composé de formule I et/ou forme éventuellement stéréoisomère du composé de formule I, où
R¹ représente
a) un atome d'hydrogène,
b) un groupe alkyle en C₁-C₆,
c) un groupe alkyle en C₁-C₄ une ou plusieurs fois substitué par l'atome de fluor, chlore, brome ou iode,
d) le groupe phényle,
e) un groupe alcényle à 2 ou 3 atomes de carbone ou
f) un groupe cycloalkyle en C₃-C₄,
R² représente
a) un atome d'hydrogène,
b) un groupe alkyle en C₁-C₄,
c) le groupe benzyle ou
d) un groupe alcényle à 2 ou 3 atomes de carbone,
R³ représente
a) un groupe pyridyle une ou plusieurs fois substitué par
1) l'atome d'hydrogène,
2) l'atome de fluor, chlore, brome ou iode,
3) le groupe nitro,
4) un groupe alkyle en C₁-C₃ ou
5) un groupe alcoxy en C₁-C₃,
b) un radical de formule II dans lequel R⁴, R⁵ et R⁶ peuvent être identiques ou différents et représentent
1) un atome d'hydrogène,
2) un groupe alkyle en C₁-C₃,
3) un groupe alkyle en C₁-C₃ une ou plusieurs fois substitué par l'atome de fluor, chlore, brome ou iode,
4) dans lequel R⁴ représente un atome d'hydrogène et R⁵ et R⁶ forment un radical méthylènedioxy,
5) un groupe alcoxy en C₁-C₃,
6) un groupe alcoxy en C₁-C₃ une ou plusieurs fois substitué par l'atome de fluor, chlore, brome ou iode,
7) un groupe alkyl(C₁-C₃)mercapto,
8) un groupe alkyl(C₁-C₃)mercapto une ou plusieurs fois substitué par l'atome de fluor, chlore, brome ou iode,
9) l'atome de fluore, chlore, brome ou iode,
10) le groupe nitro,
11) le groupe cyano,
12) un groupe alkyl(C₁-C₃)sulfonyle,
13) le groupe benzoyle,
14) un groupe benzoyle une ou plusieurs fois substitué par
14.1. l'atome de fluor, chlore, brome ou iode,
14.2. un groupe alkyle en C₁-C₃ ou
14.3. un groupe alcoxy en C₁-C₃,
15) le groupe phénoxy ou
16) un groupe phénoxy une ou plusieurs fois substitué par
16.1. un groupe alcoxy en C₁-C₃ une ou plusieurs fois substitué par l'atome de fluor, chlore, brome ou iode,
16.2. l'atome de fluor, chlore, brome ou iode,
16.3. un groupe alkyle en C₁-C₃ une ou plusieurs fois substitué par l'atome de fluor, chlore, brome ou iode,
c) un radical de formule III, et dans lequel
R¹⁰ représente
1) un atome d'hydrogène,
2) un groupe alkyle en C₁-C₄, et
n représente un nombre entier allant de 1 à 12, ou
d) R² et R³ forment ensemble, avec l'atome d'azote auquel ils sont liés, un cycle à 4-7 chaînons, qui est substitué par le groupe carbamoyle sur l'atome de carbone contigu à l'atome d'azote.

3. Composé de formule I selon la revendication 1 ou 2, dans lequel
R¹ représente un groupe alkyle en C₁-C₆,
R² représente un atome d'hydrogène,
R³ représente
a) un groupe pyridyle une ou plusieurs fois substitué par l'atome de fluor, chlore, brome ou iode, ou
b) un radical de formule II, dans laquelle R⁴, R⁵ et R⁶ peuvent être identiques ou différents et représentent
1) un atome d'hydrogène ou
2) un groupe alkyle en C₁-C₃,
3) un groupe alkyle en C₁-C₃ une ou plusieurs fois substitué par l'atome de fluor, chlore, brome ou iode,
4) dans laquelle R⁴ représente un atome d'hydrogène et R⁵ et R⁶ forment un radical méthylènedioxy,
5) un groupe alcoxy en C₁-C₃,
6) un groupe alcoxy en C₁-C₃ une ou plusieurs fois substitué par l'atome de fluor, chlore, brome ou iode,
7) l'atome de fluor, chlore, brome ou iode,
8) le groupe nitro,
9) le groupe benzoyle,
10) un groupe benzoyle une ou plusieurs fois substitué par
10.1. l'atome de fluor, chlore, brome ou iode,
10.2. un groupe alkyle en C₁-C₃ ou
10.3. un groupe alcoxy en C₁-C₃,
11) le groupe phénoxy ou
12) un groupe phénoxy une ou plusieurs fois substitué par
12.1. un groupe alcoxy en C₁-C₃ une ou plusieurs fois substitué par l'atome de fluor, chlore, brome ou iode,
12.2. l'atome de fluor, chlore, brome ou iode, ou
12.3. un groupe alkyle en C₁-C₃ une ou plusieurs fois substitué par l'atome de fluor, chlore, brome ou iode.

4. 2-cyano-3-mercapto-N-(4-trifluorométhylphényl)-crotonamide ou 2-cyano-N-(4-trifluorométhylphényl)-crotonamide-3-thiolate de sodium.

5. Procédé pour la préparation du composé de formule I, dans lequel
a) on fait réagir un cyanoacétamide de formule V 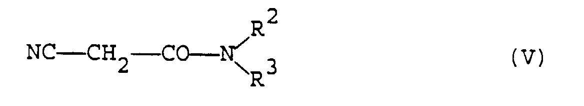 en présence d'un composé basique, avec un acide dithiocarboxylique de formule VI
R¹-CH₂-CS-S-R⁷ (VI)
R¹, R² et R³ ayant les significations données pour le composé de formule I et R⁷ représentant un groupe alkyle en C₁-C₆, ou
b) on sépare en les énantiomères purs un composé de formule I, préparé selon le procédé a), qui, en raison de sa structure chimique, apparaît sous des formes énantiomères, par salification avec des acides ou des bases sous forme d'énantiomères purs, chromatographie sur des phases stationnaires chirales ou transformation en dérivés au moyen de composés chiraux sous forme d'énantiomères purs, tels que des aminoacides, séparation des diastéréoisomères ainsi obtenus, et élimination du groupe auxiliaire chiral, ou
c) le composé de formule I, préparé selon le procédé a) ou b), est soit isolé sous forme libre, soit, dans le cas de la présence de groupes acides ou basiques, éventuellement converti en sels physiologiquement acceptables.

6. Médicament, contenant une quantité efficace d'au moins un composé de formule I selon la revendication 1 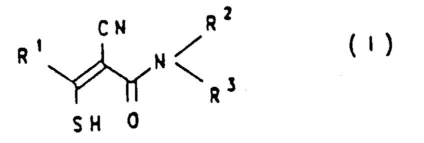 et/ou un sel physiologiquement acceptable du composé de formule I et/ou une forme éventuellement stéréoisomère du composé de formule I, les radicaux R¹, R² et R³ étant définis comme dans la revendication 1, conjointement avec un véhicule, un additif, pharmaceutiquement appropriés et physiologiquement acceptables et/ou d'autres adjuvants et substances actives.

7. Utilisation d'au moins un composé de formule I selon une ou plusieurs des revendications 1 à 4, pour la fabrication de médicaments destinés au traitement et à la prophylaxie de maladies immunitaires ou auto-immunes, de maladies à croissance cellulaire accrue, telles que le cancer ou la resténose, de réactions de rejet dans des transplantations, de maladies de peau choisies dans l'ensemble constitué par le psoriasis, le psoriasis vulgaire, le psoriasis éruptif, le psoriasis érythrodermique, le psoriasis pustuleux, la dermatite, la dermatite atopique, la dermatite allergique, la dermatite photoallergique, la dermatite médicamenteuse et les eczémas, l'asthme, l'urticaire, la rhinite, l'uvéite, le diabète de type II, la fibrose hépatique, la fibrose kystique, la colite ou l'allergie.

8. Procédé pour la fabrication d'un médicament selon la revendication 6, caractérisé en ce que l'on met sous une forme d'administration appropriée au moins un composé de formule I selon la revendication 1 et/ou un composé obtenu conformément au procédé selon la revendication 5, avec des adjuvants et véhicules physiologiquement acceptables et éventuellement d'autres additifs et/ou d'autres substances actives.
